# EUROPEAN PATENT SPECIFICATION

(11) **EP 0 474 829 B1**
(45) Date of publication and mention of the grant of the patent: **13.12.1995**
(21) Application number: 91906942.7
(22) Date of filing: 18.03.1991
(51) Int. Cl.: A61B 5/11

(54) **NON-INVASIVE METHOD FOR DETERMINING KINEMATIC MOVEMENT OF THE CERVICAL SPINE**
NICHT-INVASIVES VERFAHREN ZUR BESTIMMUNG DER BEWEGUNG DER HALSWIRBELSÄULE
SYSTEME NON INVASIF DE DETERMINATION DU DEPLACEMENT CINEMATIQUE DE LA COLONNE VERTEBRALE

(30) Priority: 30.03.1990 US 503050
(43) Date of publication of application: 18.03.1992
(73) Proprietor: FUHR, Arlan, Phoenix, Arizona 85018 (US)
(72) Inventor: FUHR, Arlan, W., Whiplash Analysis, Inc., Phoenix, AZ 85018 (US); WINTERS, Jack, Tempe, AZ 85281 (US); OSTERBAUER, Paul, Phoenix, AZ 85008 (US)
(74) Representative: Bibby, William Mark
(86) International application number: US9101796
(87) International publication number: WO9115148

(56) References cited:
- EP-A- 0 128 390
- EP-A- 0 310 901
- WO-A-87/00026
- US-A- 4 777 965

## Description

### FIELD OF THE INVENTION

The present invention relates generally to a non-invasive method for determining the kinematic function of the cervical spine. More particularly, the invention relates to a non-invasive method for determining a patient's three-dimensional (3-D) head axis of rotation during specific movements. The head axis of rotation is subsequently used in conjunction with normal data for diagnosis of abnormal kinematic function of the cervical spine due to injury and to monitor therapy administered for the treatment of neck injuries.

### BACKGROUND OF THE INVENTION

Over the years there has been considerable effort to formulate reliable methods and apparatus for measuring the movement of human body parts. Measurements of movement are made to determine if they fall within normal ranges of motion and to provide comparative information for future reference to monitor changes. For many movements, complete definition of a range of motion requires three-dimensional measurement. Spinal motion is a typical example. The kinematic function of the cervical spine has received considerable attention in recent years because of a large number of people who suffer from back pain.

Abnormal kinematic function of the cervical spine due to cervical sprain or cervical strain injuries resulting from, for example, automobile accidents, which are commonly known as "whiplash injury" afflict over 1,000,000 Americans annually. Such injuries typically involve soft tissue damage only, and a major medical as well as legal problem is to determine not only the extent of injury at the outset, but also to determine the effects of therapy and of medical treatment. Various methods of x-ray analysis (an invasive method) of the head and neck have been proposed. Only skeletal damage and major soft tissue changes can be seen with invasive techniques, and then only in the most severe cases. Computerized Tomography (CT) and Magnetic Resonance Imaging (MRI) studies have similarly been made, but they have failed to provide non-invasive, diagnostic or prognostic parameters to use as a predictor of the clinical condition.

The finite and instantaneous axis of rotation of a rigid body can be found by a variety of mathematical methods. The finite planar axis can be found by methods such as the classical Rouleaux method (e.g. Panjabi,M.M., "Centers and Angles of Rotation of Body Joints: A Study of Errors and Optimization," J. Biomechanics, Vol. 12, 1979, pp. 911-920) or the rotation matrix method of Spiegelman and Woo, S., "A Rigid-Body Method for Finding Centers of Rotation and Angular Displacements of Planar Joint Motion," J. Biomechanics, Vol. 20, 1985, pp. 715-721. These planar axes are a subset of the more general 3-D screw axis analysis but do not provide the 3-D screw axis parameters for the head-neck system.

The 3-D axis for a given finite rotation which take advantage of the x, y, and z co-ordinates of markers to the rigid body can be obtained by two mathematically-distinct methods, i) analysis based on the displacement matrix approach (e.g. Suh, C.H. and Radcliffe, C.W. Kinematics and Mechanisms Design, John Wiley & Sons, New York 1979); and ii) an approach based on minimizing error in the matrix formulation (Spoor, C.W. and Veldpaus, F.E., "Rigid body motion calculated from spatial co-ordinates of markers", J. Biomechanics, 13: 391-393, 1980; Woltring, H.J., Huiskes, R., De Lange, A. and Veldpaus, F.E., "Finite centroid and helical axis estimation from noisy landmark measurements in the study of human joint kinematics", J. Biomechanics, Vol. 18, 1985, pp. 379-389. In the first case, the algorithm uses each combination of 4 markers to estimate the screw axis parameters. Thus, with 5 markers, there are 5 solutions that in theory are the same, and with 6 markers there are 15 such solutions. The best estimate is then either the average or the median of the population. The second method utilizes all markers to estimate the appropriate information, essentially numerically solving an optimization problem. Past results suggest that the latter method is superior. Woltring, H.J., Huiskes, R., De Lange, A. and Veldpaus, F.E., "Finite centroid and helical axis estimation from noisy landmark measurements in the study of human joint kinematics", J. Biomechanics, Vol. 18, 1985, pp. 379-389.

This powerful method for determining neck kinematics function via the screw axis parameters of the head, however, has not been used in past investigations. Engineering research on the cervical spine which has resulted in a data base in which the basic parameters of cervical range of motion and static relationships of a vertebra to its adjacent vertebrae have been established to a first approximation. A limited amount of data exists in which the planar finite axis (e.g. centrode) has been found for individual vertebral rotations. Clinical Biomechanics of the Spine, White, A.A. and Panjabi, M.M. Second Edition, 1990, J.B. Lippincott, Phila., PA. These two-dimensional methods utilize cadavers or crude invasive techniques (e.g. X-rays) and, furthermore, do not give a measure of overall kinematic cervical function during head movements.

Models of whiplash injury have recently been attempted using both anthromorphic dummies and computer simulation (reviewed by Winters (1978), Sances et al. (1981)). However, these studies have considered only the general relationship between possible injury modes and crash conditions. Also, the identifying parameters are typically head acceleration and head range of motion in rotation and translation. Screw axis parameters have not been of importance. Few measurements have been made on humans. More importantly, there is little relation between measurement of the kinematics of collision using models and measurement of voluntary movements in humans. None of these "whiplash injury" studies have ever used three-dimensional kinematic screw parameters of the head as a diagnostic tool to determine the extent of abnormal kinematic cervical spine movement. See Winters J. Biomechanics; Wyss and Pollack 1981 Med. Biol. Eng. Computers; Panjabi et al., J. Biomech. 14, 1981. Thus, actual non-invasive measurements of the instantaneous or finite axis of rotation have not been used or suggested for use as a predictor or diagnostic parameter of the basic biomechanical lesion produced by the whiplash injury.

U.S. Patent Nos. 4,664,130 and 4,699,156 to Gracovetsky disclose a non-invasive method and equipment for the detection of a mechanical abnormality or injury in the lumbar spine or cervical spine of a patient and to identify this abnormality or injury as either of the compression or torsion type. In a first step, any variation of the lumbar curve of the patient is measured using a non-invasive technique. Then any discrepancy of asymmetry is detected in said measured variation of lumbar curve. Gracovetsky does not use 3-D screw-parameter kinematics of a specific rigid body (e.g. vertebrae or head), and in fact cannot obtain the 3-D axis using the method and equipment disclosed therein.

EP 0,310,901 to Gracovetsky discloses a non-invasive method and equipment for evaluating the flexibility of the spine of a patient and, based upon this evaluation, detecting and identifying possible mechanical injuries in the lumbar or cervical spine of the patient. With respect to the cervical spine, the method involves attaching markers, preferably light emitting diodes, onto the skin of the back and neck of the patient, and markers may also be attached to the back of the patient's head. The patient then flexes his head forwards and backwards and/or bends it sideways while attached to an umbilical cord or a telemetric system that connects the markers to a computer control. The markers are tracked by two spaced-apart cameras, and the 3-D coordinates of each marker are then reconstructed by computer from the data generated by the cameras. The resulting data are stated to enable measurement of scapula motion, the contribution of the cervical spine to total neck motion and the intersegmental and segmental mobility of the cervical spine.

U.S. Patent No. 4,528,990 to Knowles discloses a head-mounted apparatus for measuring the movement of the spine or head about a substantially vertical axis and is also capable of indicating spine or head tilting. A headband firmly affixed to the head includes an indicia scale used in conjunction with a body reference indicator whereby the indicator is maintained stationary while the spine or head is rotated such that the relationship between the indicator and indicia scale represents rotative body movement. A gravity operated gauge is also affixed to the head with respect to the horizontal. This device only measures orientation (angular tilt), and there is no attempt to measure axis of rotation. The same applies to Farrar, U.S. Patent No. 3,955,562 (1976).

Gilman et al. Instrumentation & Techniques, Measurement of Head Movement During Auditory Localization, Behavior Research Methods & Instrumentation, Vol.II(1), 37-41 (1979), uses a helmet apparatus with one light source marker to determine the angular position and velocity of the head in response to audio signals. The method, however, does not attempt and, in fact, cannot find the 3-D instantaneous or finite head axis of rotation. Even though Gilman et al. discloses that a 3-D system is contemplated, this addition would only allow for the determination of angular position in three axes and would still not provide axis of rotation data.

Gorron et al. also discuss the use of x-rays to calculate the instantaneous axis of rotation of the cervical vertebrae, and claim to show that a change from normal occurred in a person's centerline, indicating a dislocation of the C-7 vertebrae. Gorron, J.P., Deschamps G., Dimnet J., Fischer L.P., Kinematic Study of the Inferin Cervical Spine in Saggital Plane, pp. 32-37. In: P. Kehn & W. Widner (eds.) Cervical Spine I Springer-Verlag, N.Y. (1987). This method however, does not and is not set up to calculate the 3-D screw axis parameters of the head to provide a measure of overall cervical function.

Huntington et al., A Method of Measuring from Photographic Records the Movements of the Knee Joint During Walking, IMechE, Vol. 8, No.3 (1979) relates to a non-invasive diagnostic method and apparatus for determining real time patient ranges of motion of the knee joint by utilizing at least one video camera to track and record light reflected from markers attached to the knee joint. Huntington et al. do not disclose the use of screw axis parameters, and furthermore do not disclose a method or apparatus for use with the head-neck system.

Similar apparatus and methods have been used for study of the jaw, the back and the arm. For example, simple photography has been used to record jaw movement, and plots of the trajectory of jaw movement have been attempted. However, criteria for differentiating normal from abnormal movement have not been used, and the method is not applicable to the head-neck system.

Russian patent 904,666 discloses a device that records an observer's head position while observing an object. A screen is placed on the head of the observer and carries a two point source of light. The measuring elements of the point coordinates determines the cartesian coordinates and transmits two X, Y values to a converter which describes the movement of the two points and hence the movement of the head. By increasing the number of screens and recorders the general case with 3 dimensions can be handled. There is no teaching to obtain screw parameters or to utilize such information as a diagnostic tool.

Berger U.S. Patent No. 4,586,515 discloses a device and method for measuring the position and/or motion of a body part, and particularly the head to diagnose spinal disorders. Three sensors are used to detect three-dimensional motion of the head. Rotation, flexion and lateral tilting of the head are detected by the device to determine the motion pattern of the body part in space to diagnose a motion disorder. Berger does not use biomechanical screw axis pathways to determine the nature and extent of abnormal head movement.

Thus, despite the various attempts of those skilled in the art, the art has failed to develop a reliable method for determining abnormal kinematic movement of the cervical spine. More particularly, the art has failed to recognize the use of the 3-D kinematic screw axis parameters of the head as an indicator of cervical spine function, and, thus, has failed to provide a satisfactory non-invasive method for using the 3-D kinematic screw axis of the head-neck system as a diagnostic tool to determine the nature and extent of abnormal kinematic cervical spine movement.

Accordingly it is a principal object of the present invention to provide a non-invasive method for using 3-D kinematic screw axis parameters as a diagnostic tool to determine the nature and extent of abnormal kinematic cervical spine movement.

It is a more specific object of this invention to provide biomechanical, numerical parameters by which to establish abnormal kinematic function of the cervical spine which occurs in patients who suffer "whiplash injury."

Yet another object of the invention is to provide a procedure necessary to carry out the above-identified methods.

These and other advantages of the invention as well as additional inventive features will become apparent from the following detailed description of a preferred exemplified embodiment of the invention and accompanying drawings.

### SUMMARY OF THE INVENTION

The present invention is predicated on the discovery that the kinematic movement of a body part provides biologically relevant information about the pathway through which the body part rotates. The biomechanical pathway, in turn, provides a means by which to compare physically impaired movement with normal or standard movement for the diagnosis and prognosis of physical injury to the body part. The present invention is particularly useful with the head-neck system. The instantaneous axis of rotation vector, centrode and/or finite rotation pole of the head define the biomechanically relevant pathway of the head's movement. Because the head is the largest member of the kinematic chain of the head-neck system, the biomechanical pathway of the head may be used to diagnose kinematic abnormalities in the cervical spine.

The present invention provides a non-invasive 3-D method for detecting the kinematic function of the cervical spine of a patient and can be used to detect abnormal kinematic functions as well. By the method of the present invention, the axis of rotation of the head is used to determine neck function by finding the correct level of this rotation and any coupled motion.

According to the invention there is provided a non-invasive 3-D method for determining the kinematic function of the cervical spine of a human patient, comprising the steps of:
a. positioning recordable, detectable marker means onto the head of the patient;
b. using a target or instructional means to guide the patient through spatial head movements;
c. recording the positions of said marker means as the patient moves his head in response to said target or instructional means;
d. processing the recorded positions of the marker means by a mathematical rigid body analysis means to derive screw axis parameters of the patient's head which define a 3-D instantaneous and, optionally, a finite axis of rotation of the patient's head;
e. using the axis of rotation of said patient's head as a measure of cervical kinematic function during head movements; and
f. comparing the derived head axis of rotation for said patient either with a head axis of rotation for predetermined, standardized data to ascertain any discrepancy or with a previous derived head axis of rotation for said patient to identify changes.

### BRIEF DESCRIPTION OF THE DRAWINGS

FIGURE 1 is a perspective view of equipment suitable for carrying out the method of the present invention;
FIG. 2 is a perspective view of a helmet to be worn by the patient to provide marker means and pointer means;
FIG. 3 is a schematic view of a target means comprising lamps which can be selectively illuminated to cause the desired head movements by the patient;
FIG. 4 is a perspective view of a target means in a generally dome-shaped structure;
FIG. 4a is an end view of the interior of the dome-shaped target;
FIG. 4b is a schematic of the location of the signal means of the target means for a planar target means and for a dome-shaped target means;
FIGS. 5 and 6 are schematic views of the target means illustrated in Fig. 3 wherein various preselected patterns for the range of motion analysis have been identified;
FIGS. 7a and 7b (after 6 weeks) illustrate 3-D plots of the derived finite axis of rotations for flexion-extension in 10° intervals for a normal patient.
FIGS. 8a and 8b (after 6 weeks) illustrate 3-D plots of the derived finite axis of rotations for flexion-extension in 10° intervals for patients afflicted with a whiplash injury.

### DETAILED DESCRIPTION OF THE PREFERRED EMBODIMENTS

In accordance with the present invention kinematic function of the cervical spine is detected by recording means, which record the patient's 3-D movements. The recording means record a signal from marker means which are radially disposed about a helmet worn by the patient, as the patient moves his head according to the target means. The target means is comprised of a plurality of signal emitting means, preferably lamps or light emitting diodes. The signal emitting means are suitably controlled by a control means which causes the signal emitting means to emit light in a preselected pattern. The patient is instructed to follow the signal generated by the target means by moving his head in response to the emitted signal from the target means. In so doing, the patient moves his head in the selected pattern and his head moves in three dimensions in space. As the patient follows the target signals, his head's movement is recorded by recording means which detect and record the movement of the marker means. With the use of a 3-D spatial analysis system, the 3-D coordinates of each of the marker means is determined. Using rigid body kinematic principles, kinematic parameters defining the "screw axis" about which the head is rotated are obtained. The derived screw axis parameter data for the patient may then be compared to standardized screw axis parameter data to determine the kinematic function of the patient's cervical spine.

While we do not wish to be bound to any particular theory, it is believed that an analysis of the largest rigid body of the head-neck system, namely the head, will provide information that will be indicative of the kinematic function of the cervical spine. That theory is predicated on the fact that the head is at the end of a kinematic chain of rigid bodies that are intricately coupled, so that details of a given head orientation will depend on cervical spine function. For example, if the C1-C2 region cannot rotate normally, the screw axis of the head will be lower. Conversely, injury limiting the range of motion in the C5-C7 area will likely cause the axis to be higher for a given type of task.

The present invention provides a real time fully 3-D quantitative analysis of the head-neck system by evaluating the cervical biomechanical pathway of the head during natural head movements. Deviations in the biomechanical pathway of the person under test from standardized data, or normal, are indicative of abnormal movement of the cervical spine, and in turn indicate injury to the spine. Thus, by observing the biomechanical pathway of the patient under test, asymmetries anywhere in the cervical spine can be immediately identified. Moreover, because of wide variability in the screw axis vector directions, more abnormalities may be measured using the method of the present invention as compared to any prior means. For example, movements such as axial rotation and lateral flexion are in fact often more difficult for individuals with whiplash injuries, which typically involve soft tissue damage. Subtle changes in the screw axis parameters of individuals with whiplash injury are measurable in accordance with the present invention and can be used satisfactorily as clinical evaluators, without invasive methods and without knowledge of the pathomechanical cause of the injury.

Accordingly, of primary interest in the method of the present invention are the 3-D screw axis parameters for the head of a human patient. The screw axis parameters for the head consist of (a) the axis of rotation vector, (b) a point on this vector, and, as appropriate, (c) the angle of rotation about this vector and sliding along this vector and (d) the angular velocity. The biomechanical pathway of the head through its movement consists of the instantaneous axis of rotation, the centrode pathway, and the finite rotation pole. By calculating the biomechanical pathway, preferably with the aid of a computer, clinically reliable data can be obtained from which the degree of abnormal kinematic function can be ascertained. Desirably, the various elements of the biomechanical pathway are plotted graphically to facilitate comparison with the biomechanical pathways of standardized data, which may comprise, for example the biomechanical pathways of a population of normal individuals, i.e. those who have not been injured, and/or with the patient's prior history. The method of the present invention thus provides a significant diagnostic and prognostic tool for determining the nature and extent of abnormal kinematic movement of the cervical spine.

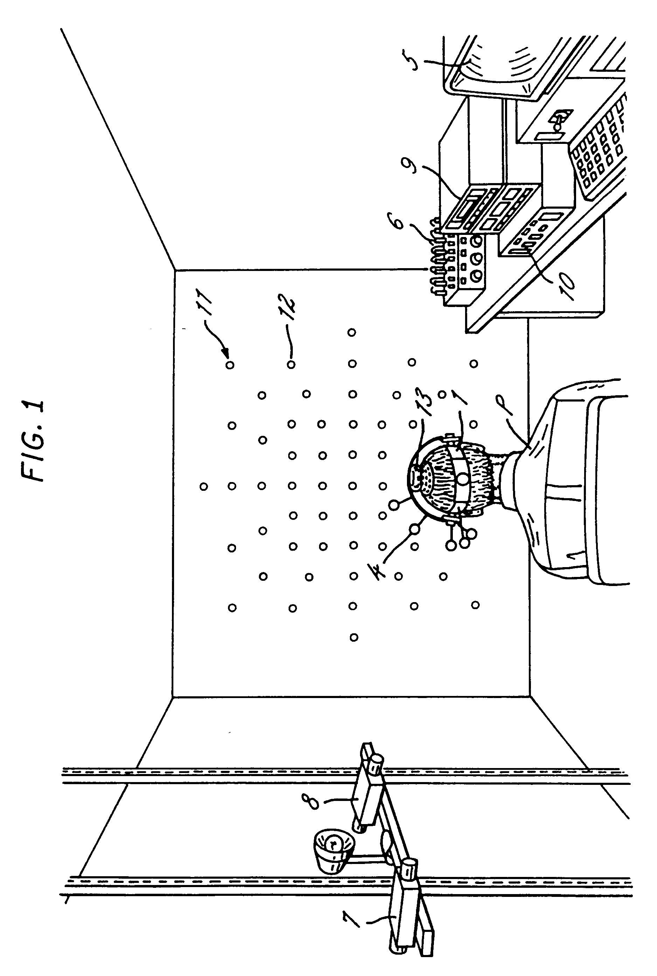

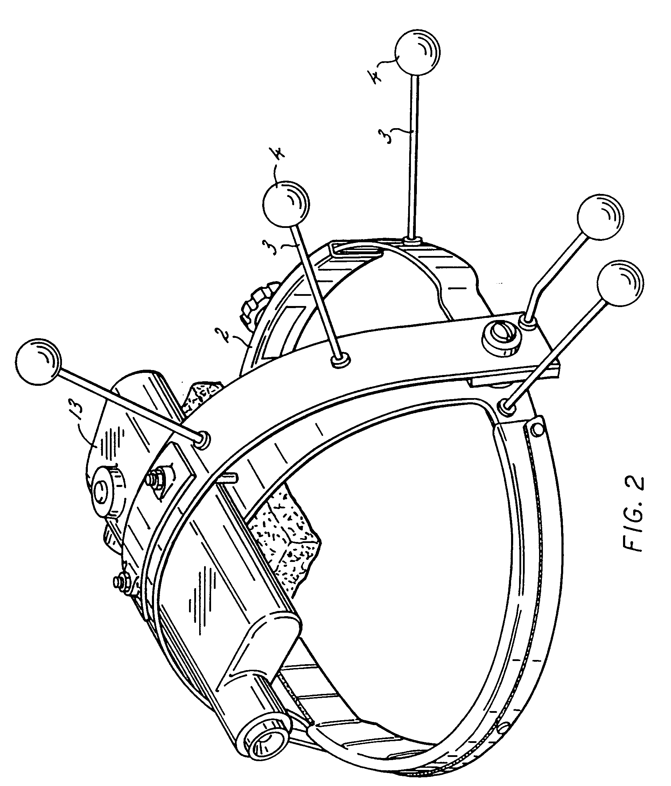

Turning to FIG. 1, there is shown equipment suitable for carrying out the method of the present invention. According to the illustrated embodiment a helmet 1 is worn by the patient P. The helmet 1 can be adapted to various head sizes and shapes by means of an adjustable strap 2 (FIG. 2); and is preferably lightweight so that it can fit comfortably on the head. To carry out the testing (described in more detail hereinafter) the helmet is adjusted to fit snugly on the patient's head during the test. Thus movement of the helmet will accurately reflect the patient's head movements.

The helmet 1 includes marker means which can be detected by a recording means to enable the determination of the patient's head orientation in space. The size and shape of the marker means is not critical to the method of the invention provided that the marker means are capable of being detected and recorded by the recording means. In the illustrative embodiment, the marker means comprise bolts 3 of variable length mounted to helmet 1 and preferably include on their ends a marker 4 which is capable of reflecting light. Preferably, the markers 4 comprise a 1.27 cm (one-half inch) diameter spherical ball covered by a suitable reflective material, preferably glass beads. Preferably five or six bolts are mounted to the helmet in such a manner that all of the markers can be uniquely recorded by each recorder means without any marker overlap or marker interference, one with another, throughout the patient's range of motion.

The equipment for use in the present invention further includes a target means which comprises a plurality of signal emitting means in preselected arrangement. The signal emitting means are capable of being selectively activated to emit a signal to be detected by the patient. The patient tracks the target signal and in so doing, moves his head to follow the pattern that is created.

In accordance with a preferred embodiment of the present invention, the signal emitting means are controlled so that the signal means can be activated in a preselected pattern, consonant with a specific head movement sought to be tested. Thus, for example, the signal means may be activated in a manner such that the range of motion of the patient's head will be tested for axial rotation (i.e. left-to-right movement) flexion-extension (up and down movement) or lateral bending (side-to-side movement) or an oblique movement, such as an x-shaped pattern, a box-shaped pattern, or the like. Preferably, the signal emitting means comprises a plurality of light sources each of which can be selectively controlled to light up in any desired sequence or pattern. The light source for indicating the tracking direction which the patient is to follow is preferably a lamp, or a laser.

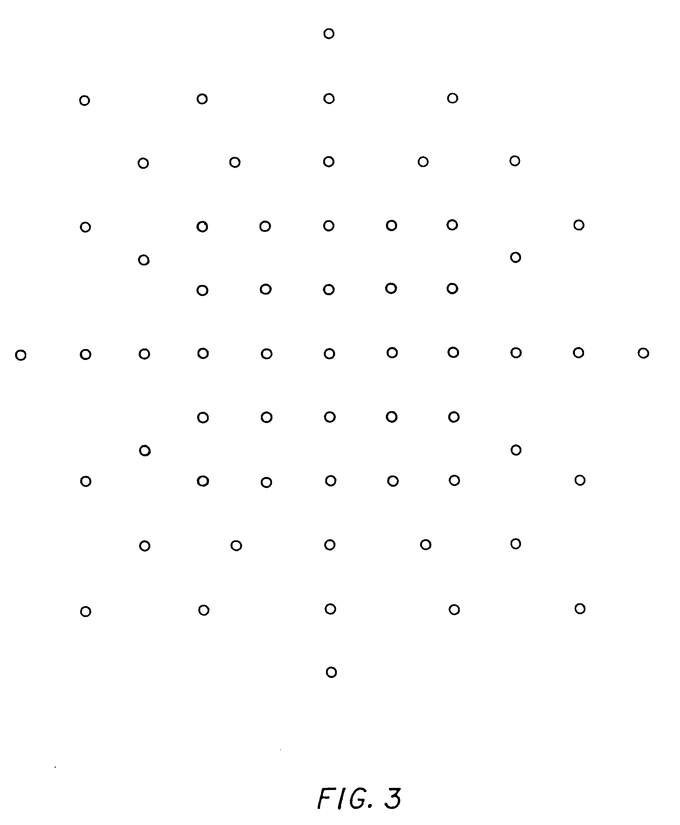

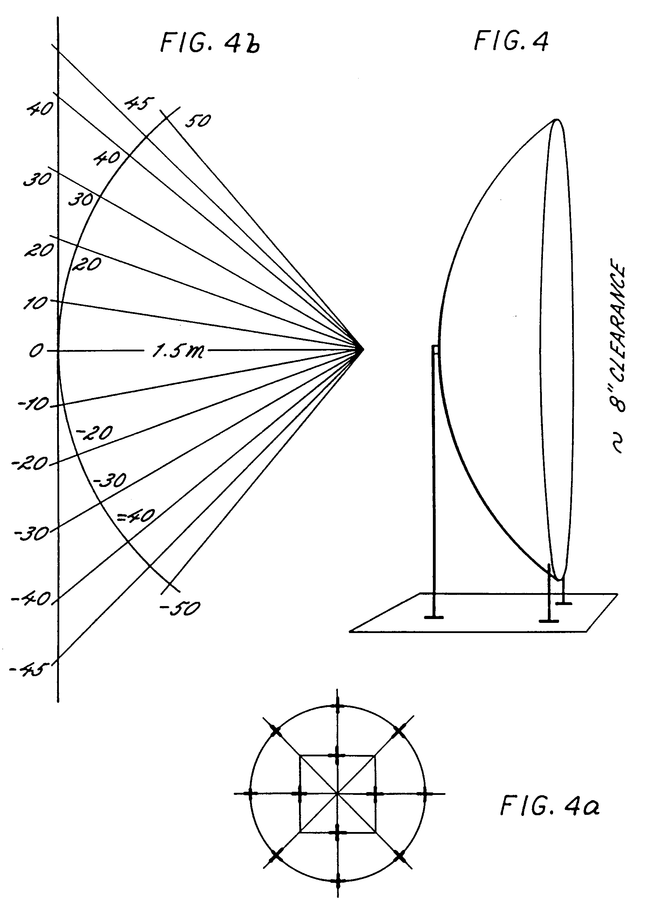

In the illustrative embodiment shown in Fig. 1, the target means comprises a fixed planar array or grid 11 of small lamps 12, each just under 1.27 cm (1/2 inch) in diameter. The grid is arranged so that the total viewing angle of the patient from the far left side of the grid to the far right side of the grid is about 100°, and so that the viewing angle from the top of the grid to the bottom of the grid is likewise about 100° (Fig. 3). The viewing angles to the left of center, to the right of center, to the top from center and to the bottom from center are all about 50°. In addition, the lamps in the grid are set apart by an incremental viewing angle of approximately 10° (Fig. 3). In the illustrative embodiment, the grid comprises sixty-one lamps (Fig. 3). Alternatively, the lamps may be oriented in other configurations, using the same or similar angular relationship between lamps. For example, the lamps may be configured in a dome-shaped or umbrella-shaped structure. Fig. 4 illustrates an easily erectable portable dome-shaped target means 20 supported by legs 21 mounted to a base 22. The portable dome-shaped target means can be moved easily and, due to its smaller overall dimension as contrasted with a planar-shaped target means, it may be used in a smaller area than a planar target. An exemplary pattern of the lamps of the target means is shown in Fig. 4a which is an end view of the grid looking into the dome from a point X outside the dome. In the dome-shaped target means shown in Fig. 4 the lamps are located in the same angular relationship as they are in a planar target means, as can be seen in Fig. 4b. With the dome-shaped target means 20 the lights are all equidistant from the patient.

The lamps in the grid are selectively controlled so that they can be activated in any preselected pattern desirable. To that end, and in the illustrative embodiment, the lamps in the grid are controlled by a suitable central processing unit, such as, for example, a personal computer 5, an interface board (not shown) for the central processing unit, an optocoupler box 6, and the appropriate wiring (not shown) to connect the lamps to the other components. The optocoupler box converts low-power computer output signals into higher-power signals capable of turning on the grid lamps in the appropriate sequence. The optocoupler box 6 may be a personal computer, computer interface board and appropriate optocoupler circuitry, or other appropriate electronic circuitry for creating the desired light sequences. The central processing unit interface board may be, for example, a board commercially available from Metrabyte as DDA-6. Commercially available data acquisition and display software, such as, for example, Lab Tech Notebook available from Laboratory Technologies may be used as the medium to set up a pre-programmed sequence of light patterns to elicit the desired range of motion.

In the preferred embodiment, sixteen lamps are controlled with the digital I/O channels that are available on the computer interface board. While the computerized system for controlling the grid lamps provides considerable flexibility, it will be appreciated that other types of control may also be suitably employed. Thus, an alternative to the computerized control system is the use of electronic switching circuitry. The electronic switching circuitry can be manually operated with push-buttons or the like to create a certain preselected light sequence.

In one embodiment of the present invention, helmet 1 includes an indicator means 13. The indicator means cooperates with the target means to indicate whether the patient has moved his head from one lamp to the next as the lamps are lit in the desired pattern. It will be appreciated by those skilled in the art that one of the indicator means or the target means may be selected to emit a signal and the other selected to receive that signal to provide confirmation that the patient's head moved in accordance with the prescribed pattern. In accordance with a preferred embodiment of the present invention, the indicator means 13 emits a beam of light which is to be directed at the target and detected on or by the target. In carrying out the method of the present invention, as the patient follows the target signals as they are activated he moves his head so that the beam from the indicator means illuminates the activated target signal lamp. Illumination of the target signal lamp in this manner provides visual confirmation that the patient has moved his head in response to the target signal and thus is following the prescribed pattern.

In accordance with a preferred embodiment of the invention, the recording means comprise at least two standard CDC video cameras, 7 and 8, which record the position of the marker means throughout the preselected range of motion tasks the patient is asked to perform. The two cameras are placed relative to the patient's head so that all the markers 4 on the helmet 1 will be within the camera's recording view for the entire range of the patient's expected motion. Generally the cameras are set at an angle of 50° to 60° relative to one another. However, the angle at which the cameras are located relative to one another is not critical provided they are not too close together, which may reduce accuracy, and provided that each camera can cover the entire range of expected motion and can continue to view all marker means and record their movement. In the preferred embodiment, the video recorders record light reflected by the marker means carried by the helmet, which corresponds to the patient's head movement in space.

Each video camera is connected to a video cassette recorder 9, 10, where a videotape of the patient's motion is recorded on videocassette. A monitor (not shown) may be used to view the video camera images as they are being recorded. In order to minimize unwanted reflection artifacts from the videorecording, it is preferred that all of the helmet elements other than the markers 4 be of a non-reflective color such as dark blue or black.

In order to provide an accurate three-dimensional analysis of the patient's head movements, the two videotapes are first synchronized with an "Expert Vision Remote Site" unit, commercially available from Motion Analysis, Inc. The Expert Vision Remote Site unit synchronizes the camera sampling which is done every 17 ms via a signal from the remote site to each camera, and places a user initiated audio tone on each videotape at the same sampling frame. The remote site unit is connected to the VCR which is hooked up to the two video cameras so that the timing marks are created simultaneously with the actual filming of the patient.

In accordance with the invention, the 3-D coordinates of the centroids of the markers 4 throughout the movement of the head during the patient's performance of the requested tracking tests are used to compute the finite rotation pole, the instantaneous axis of rotation and the centrode movement of the head. To obtain the 3-D coordinates of the centroid of each marker 4 from the videotape, the full "Expert Vision Motion Analysis System," commercially available from Motion Analysis, Inc., Santa Rosa, Ca., is preferably used. This system comprises a three-dimensional motion analysis system housed on a SUN computer workstation and a master software program called "EV3d". While certain standard commands from the Expert Vision System are utilized for calibration and initialization, the special command "vide" is used to create computer files from videotape. The "vide" command couples the computer to Motion Analysis' "VP-110" hardware, or its equivalent, which measures the grey-level transition, which is essentially an outline of the markers 4, from a videotape. The "track" command of the Expert Vision System allows the computer files created from each videotape to be combined and the centroid or location for each marker throughout the head movement and in three-dimensions to be obtained for each motion task the patient performs. Other photoelectronic or electro-magnetic-based systems may likewise be used.

After the marker locations have been determined, the screw axis parameters are calculated, preferably with the aid of a computer. To that end, software based on the equations first presented by Spoor, C.W. and Veldpaus, F.E., "Rigid body motion Calculated from spatial co-ordinates of markers", J. Biomechanics, 13: 391-393, 1980, and as presented in Woltring, H.J., Huiskes, R., De Lange, A. and Veldpaus, F.E., "Finite centroid and helical axis estimation from noisy landmark measurements in the study of human joint kinematircs", J. Biomechanics, Vol. 18, 1985, pp. 379-389, may be used satisfactorily. Marker locations in addition to the four typically utilized by clinical theoretical techniques are advantageously used to lower the error in the calculation due to measurement noise. Using the equations of Spoor and Veldpaus and Woltring, et al., the screw axis parameters or biomechanical pathway for the head, namely the instantaneous axis of rotation, a point on this axis (e.g. the finite rotation pole, or centrode) and the rotation about and sliding along this axis can be obtained. Plots of these various movements and pathways may then be made by standard graphics methods.

Graphic plots of the derived screw axis parameters for the patient under test may then be used in either of two ways. The derived screw axis parameters may be used as a diagnostic tool-by comparing the screw axis parameters with standardized screw axis parameters, including, for example, plots of screw axis parameters for normally healthy persons, to ascertain any discrepancies between the two. In that way, any abnormal kinematic movement of the cervical spine can be observed and quantified so that the degree of injury can be established.

Therapeutic changes (e.g., improvement) in the kinematic function of the cervical spine also can be evaluated quantitatively by comparing the derived screw axis parameters of the patient after therapy to the analog of the screw axis parameters derived for that patient before therapy was begun. It will be readily apparent to those skilled in the art that the use of the derived screw axis parameters data after injury combined with the screw axis parameters measured periodically after treatment will provide quantitative information regarding the patient's progress after therapy and the relative degree of impaired kinematic function from which the patient still suffers.

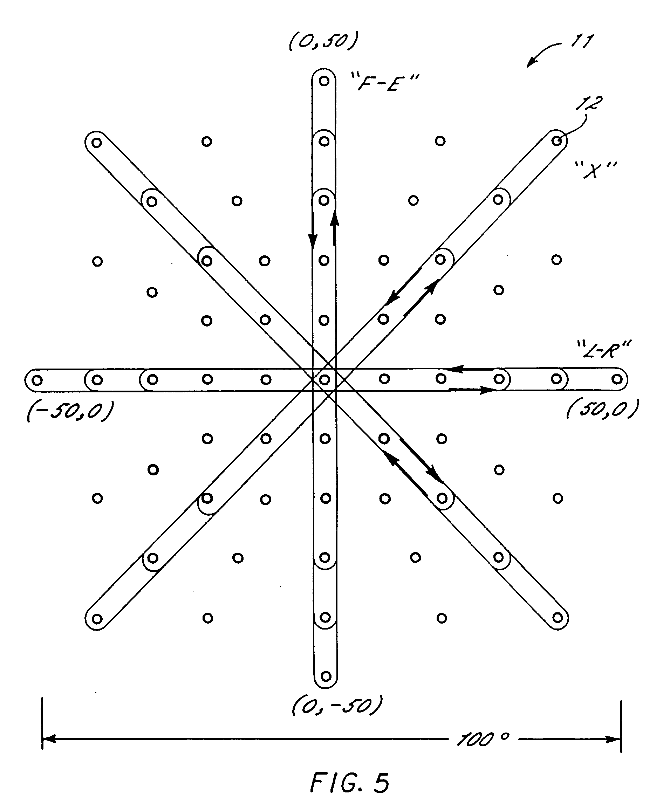

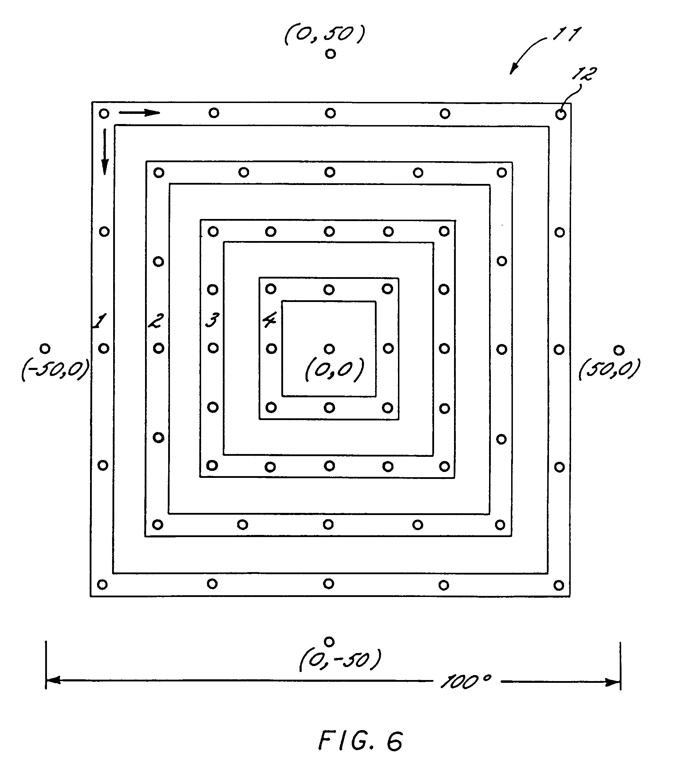

In order to carry out the method of the present invention, a patient P is fitted with the helmet 1 described above. The patient is then instructed to perform a series of voluntary range-of-motion tasks to provide the outside parameters of his range of motion for the actual test. More particularly, the patient starts by making standard, voluntary, self-paced slow range-of-motion movement in the flexion-extension (up-down) axial rotation (left-right) and lateral bending (side-to-side) directions. Following the voluntary range-of-motion tasks, the patient is instructed to follow target patterns within his voluntary range of motion. Preferably five target sequences are used: left-right; (Fig. 5) up-down (Fig. 5) a box pattern (Fig. 6) in either direction and an oblique or x-shaped pattern (Fig. 5). While the patient is following the target pattern, movement of the markers on the helmet (which correspond to head movement) are recorded on the synchronized videocassette tapes for further processing as described heretofore. The data recorded on the videotapes are then processed as described above. From that data which corresponds to the head's movements, the screw axis parameters are calculated and plotted. The derived plots are then compared to standard plots and/or to prior plots of the same patient so that the nature and/or extent of the abnormal kinematic movement of the cervical spine can be determined.

The following Examples are intended to further ilustrate the invention described herein, and are not intended to limit the scope of the invention.

### EXAMPLE I

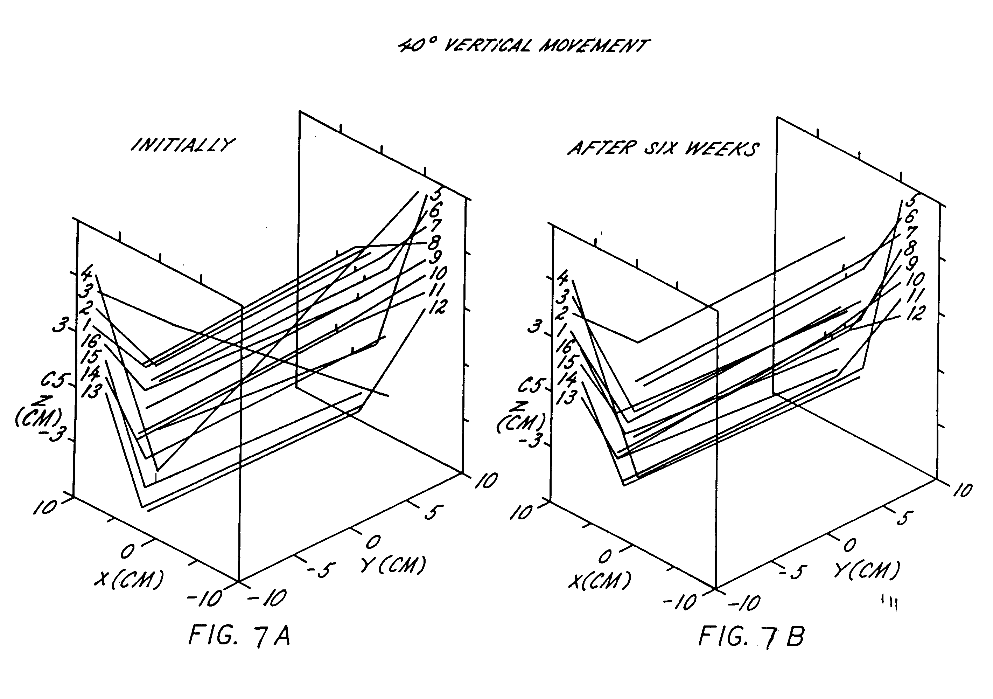

A normal patient, that is, one not afflicted with whiplash injury, was tested for 3-D head movement using the apparatus and method described heretofore. The patient's flexion-extension (vertical movement) was measured at 10° intervals over 40°. The patient was tested using the target means illustrated in FIG. 3 and the five target sequences described above. The screw axis for vertical movement was then calculated and plotted. The finite axis of rotation (defined by screw axis parameters) is plotted, expressed in centimeters, relative to the fifth cervical vertebrae (C5) as illustrated in Figure 7a. The patient was then re-tested after six weeks (Figure 7b) to determine the consistency of the movement of the patient over that time interval.

It can be seen from this data that the screw axis parameters of a normal patient vary smoothly in location and orientation. That smoothness in location and orientation of the movement was to be expected for a normal patient. In fact, the average (n = 9) finite axis crossing for the entire normal population during 10° interval vertical movements was found to vary smoothly between the third cervical and first thoracic vertebrae. This example demonstrates the efficacy of the method and apparatus of the present invention to provide both the screw axis parameters of the head's movement and the mathematical values of such movement, the latter of which provide a complete description of the character of that movement. Accordingly, the method and apparatus of the present invention allow, for the first time, both the calculation of the screw-axis parameters of the head-neck system and a mathematical analysis of that movement for diagnostic and prognostic evaluation.

### EXAMPLE II

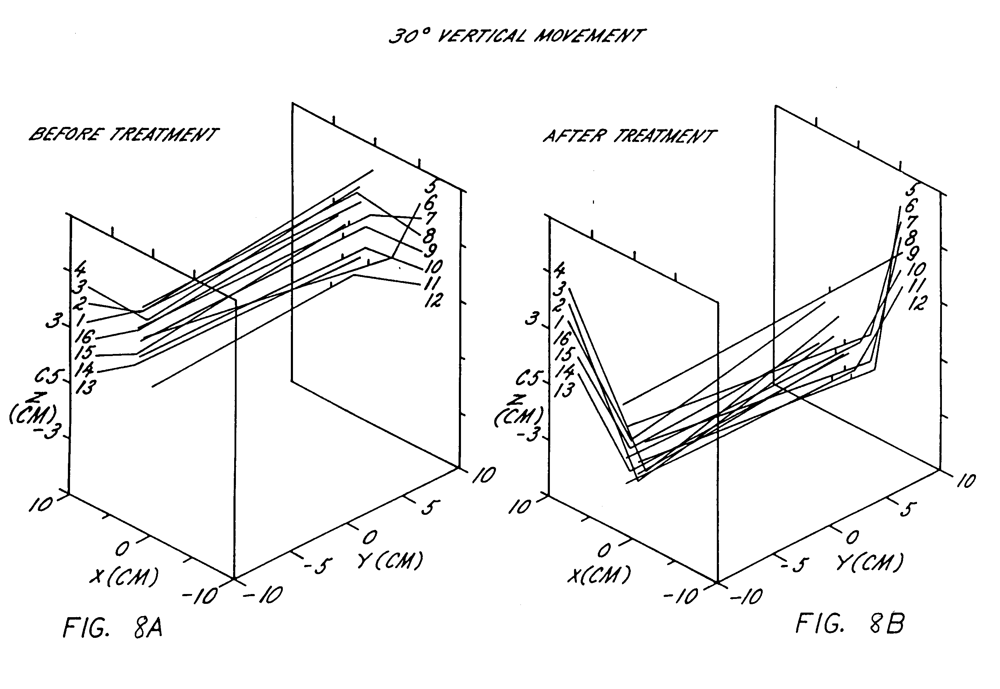

A patient afflicted with whiplash injury was tested for 3-D head movement in the same manner that the normal patient of Example I was tested. The patient's flexion-extension was measured at 10° intervals over 30°. The finite axis of rotation (defined by the screw axis parameters) for flexion-extension was then calculated and plotted for the patient as illustrated in Fig 8a. These screw axis parameters were plotted in different planes expressed in centimeters, relative to the fifth cervical vertebra (C5). The patient was then re-tested six weeks later (Fig. 8b) after undergoing treatment for the whiplash injury.

It can be seen that for the patient with whiplash injury the screw axis location on the z-plane differs from that of the normal population. Before treatment, the patient tends to make all rotations about the upper cervical vertebrae (median axis approximately at the third cervical level), which may indicate problems in the lower neck region. After six weeks of treatment, the finite axis location on the z axis has shifted toward a more normal distribution, with the median axis located at approximately the fifth cervical vertebra.

## Claims

1. A non-invasive 3-D method for determining the kinematic function of the cervical spine of a human patient, comprising the steps of:
a. positioning recordable, detectable marker means onto the head of the patient;
b. using a target or instructional means to guide the patient through spatial head movements;
c. recording the positions of said marker means as the patient moves his head in response to said target or instructional means;
d. processing the recorded positions of the marker means by a mathematical rigid body analysis means to derive screw axis parameters of the patient's head which define a 3-D instantaneous and, optionally, a finite axis of rotation of the patient's head;
e. using the axis of rotation of said patient's head as a measure of cervical kinematic function during head movements; and
f. comparing the derived head axis of rotation for said patient either with a head axis of rotation for predetermined, standardized data to ascertain any discrepancy or with a previous derived head axis of rotation for said patient to identify changes.

2. A non-invasive 3-D method for determining the kinematic function of the cervical spine of a human patient, as claimed in claim 1 comprising the additional step of storing the recorded positions of the marker means prior to the processing step.

3. A non-invasive 3-D method for determining the kinematic function of the cervical spine as claimed in claim 1 or claim 2 when the processing step includes the steps of processing the recorded positions of the marker means by 3-D analysis to yield 3-D information of the marker means, and processing the 3-D information of the marker means by said mathematical rigid body analysis means.

## Patentansprüche

1. Nicht-invasives, dreidimensionales Verfahren zum Bestimmen der kinematischen Funktion der Halswirbelsäule eines menschlichen Patienten, das die folgenden Schritte umfaßt:
a) Anbringen aufzeichenbarer, nachweisbarer Markierungseinrichtungen am Kopf des Patienten;
b) Einsatz einer Ziel- bzw. Anleiteinrichtung, um den Patienten durch räumliche Kopfbewegungen zu führen;
c) Aufzeichnen der Positionen der Markierungseinrichtungen, wenn der Patient seinen Kopf in Reaktion auf die Ziel- bzw. Anleitungseinrichtung bewegt;
d) Verarbeiten der aufgezeichneten Positionen der Markierungseinrichtungen mit einer mathematischen Starrkörper-Analyseeinrichtung, um Schraubenachsenparameter des Kopfes des Patienten herzuleiten, die eine dreidimensionale Momentan- und wahlweise eine finite Drehachse des Kopfes des Patienten definieren;
e) Verwenden der Drehachse des Kopfes des Patienten als ein Maß der kinematischen Halsfunktion bei Kopfbewegungen; und
f) Vergleichen der hergeleiteten Kopfdrehachse für den Patienten entweder mit einer Kopfdrehachse für vorgegebene standardisierte Daten, um mögliche Abweichungen festzustellen, oder mit einer zuvor hergeleiteten Kopfdrehachse für den Patienten, um Veränderungen zu erkennen.

2. Nicht-invasives, dreidimensionales Verfahren zum Bestimmen der kinematischen Funktion der Halswirbelsäule eines menschlichen Patienten nach Anspruch 1, das den zusätzlichen Schritt des Speicherns der aufgezeichneten Positionen der Markierungseinrichtungen vor dem Verarbeitungsschritt umfaßt.

3. Nicht-invasives, dreidimensionales Verfahren zum Bestimmen der kinematischen Funktion der Halswirbelsäule nach Anspruch 1 oder Anspruch 2, wenn der Verarbeitungsschritt die Schritte des Verarbeitens der aufgezeichneten Positionen der Markierungseinrichtungen durch dreidimensionale Analyse umfaßt, um dreidimensionale Information der Markierungseinrichtungen zu erhalten, sowie des Verarbeitens der dreidimensionalen Information der Markierungseinrichtungen mit der mathematischen Starrkörper-Analyseeinrichtung.

## Revendications

1. Méthode 3-D non invasive pour déterminer la fonction cinématique de la colonne vertébrale d'un patient humain, comprenant les étapes :
a. de positionnement de moyens marqueurs détectables, enregistrables, sur la tête du patient,
b. d'utilisation de moyens de voyant ou d'instruction pour guider la patient à travers des mouvements de tête dans l'espace,
c. d'enregistrement des positions des moyens marqueurs lorsque le patient déplace sa tête en réponse aux moyens de voyant ou d'instruction,
d. de traitement des positions enregistrées des moyens marqueurs par des moyens d'analyse mathématique de corps rigide pour dériver des paramètres d'axe hélicoïdal de la tête du patient qui définissent un instantané en 3-D et éventuellement un axe de rotation fini de la tête du patient,
e. d'utilisation de l'axe de rotation de la tête du patient comme mesure d'une fonction cinématique cervicale pendant des mouvements de tête, et
f. de comparaison de l'axe de rotation de la tête dérivé pour ledit patient soit avec un axe de rotation de tête pour des données standardisées, prédéterminées, en vue pour constater une différence quelconque, soit avec un axe de rotation de tête précédemment dérivé pour ledit patient pour identifier des changements.

2. Méthode 3-D non invasive pour déterminer la fonction cinématique de la colonne vertébrale d'un patient humain, suivant la revendication 1, comprenant l'étape additionnelle de mémorisation des positions enregistrées des moyens marqueurs avant l'étape de traitement.

3. Méthode 3-D non invasive pour déterminer la fonction cinématique de la colonne vertébrale, suivant l'une des revendications 1 et 2, dans laquelle l'étape de traitement comprend les étapes de traitement des positions enregistrées des moyens marqueurs par analyse en 3-D pour fournir une information en 3-D des moyens marqueurs et de traitement de l'information en 3-D des moyens marqueurs par les moyens d'analyse mathématique de corps rigide.
